(19) Europäisches Patentamt
European Patent Office
Office européen des brevets

(11) Veröffentlichungsnummer: **0 032 576**
**B1**

(12) # EUROPÄISCHE PATENTSCHRIFT

(45) Veröffentlichungstag der Patentschrift: **10.08.83**

(21) Anmeldenummer: **80108099.5**

(22) Anmeldetag: **22.12.80**

(51) Int. Cl.³: **C 07 C 33/20,** C 07 C 47/228, C 07 C 47/235, C 07 C 69/025, C 07 C 29/32, C 07 C 67/28

(54) **Verfahren zur Herstellung von p-substituierten Phenylpropanolen und deren Estern sowie von p-substituierten 3-Phenyl-2-methyl-propanalen.**

(30) Priorität: **29.12.79 DE 2952719**

(43) Veröffentlichungstag der Anmeldung: **29.07.81 Patentblatt 81/30**

(45) Bekanntmachung des Hinweises auf die Patenterteilung: **10.08.83 Patentblatt 83/32**

(84) Benannte Vertragsstaaten: **CH DE FR LI NL**

(56) Entgegenhaltungen:
**EP-A-0 004 881**
**DE-B-1 144 727**
**DE-B-1 227 882**
**DE-B-2 417 357**
**GB-A-796 739**
**US-A-3 739 039**
**BEILSTEINS HANDBUCH DER ORGANISCHEN CHEMIE, 4. Auflage, Band 7, 1925, VERLAG JULIUS SPRINGER, Berlin Seite 369**
**Chemical Abstracts Band 60, Nr. 11, 25. Mai 1965 Columbus, Ohio, USA S. ABE et al. «Cyclamen-type perfume compounds. II. Alkyl-substituted derivatives of hydrocinnamaldehyde» Spalte 13175 g-h**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Hoffmann, Werner, Dr., Ringstrasse 11 C, D-6701 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

Verfahren zur Herstellung von p-substituierten Phenylpropanolen und deren Estern sowie von p-substituierten 3-Phenyl-2-methyl-propanalen

Die Erfindung betrifft ein Verfahren zur Herstellung von in p-Stellung substituierten Phenylpropanolen bzw. deren Estern der allgemeinen Formel I

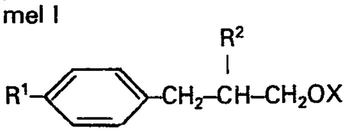

(I)

in der $R^1$ einen Alkylrest mit 3 bis 10 C-Atomen oder einen Cycloalkylrest mit 5 bis 7 C-Atomen, $R^2$ H oder einen Alkylrest mit 1 bis 4 C-Atomen und X Wasserstoff oder einen Acylrest mit 2 bis 10 C-Atomen bedeuten, durch Umsetzen von Phenylpropanolen bzw. Estern von Phenylpropanolen mit Olefinen oder Alkylhalogeniden in Gegenwart von bestimmten Friedel-Crafts-Katalysatoren sowie ein Verfahren zur Herstellung der als Riechstoffe interessanten p-substituierten 3-Phenyl-2-methyl-propanalen ausgehend von Benzaldehyd über die Phenylpropanole der Formel I.

Die Bedeutung des erfindungsgemässen Verfahrens besteht darin, dass man aus einfachen Phenylpropanolen bzw. deren Estern durch Umsetzung mit den entsprechenden Olefinen bzw. Alkylhalogeniden auf leichte und bequeme Weise zu in p-Stellung substituierten Phenylpropanolen bzw. deren Estern gelangt, die als Derivate des bekannten Riechstoffes Hydrozimtalkohol (3-Phenyl-propan-1-ol) als Geruchsstoffe von Interesse sind. Diese substituierten Phenylpropanole können nach bekannten Methoden durch Dehydrierung an Kupfer- oder Silberkatalysatoren bzw. durch Oxidation mit Chrom-IV-Verbindungen (vgl. Houben-Weyl «Methoden der organischen Chemie», Band 7/1, S. 160ff, S. 171 f) in die entsprechenden Aldehyde überführt werden. Unter diesen Aldehyden gibt es einige Vertreter, wie den Cyclamenaldehyd (3-p-Isopropylphenyl-2-methylpropanol) oder das Lilial (3-p-Tertiärbutylphenyl-2-methyl-propanol), die bekannte und gleichzeitig sehr gesuchte Riechstoffe sind. Die Synthese dieser beiden Substanzen erfolgte bisher aus den technisch relativ schwer zugänglichen substituierten Benzaldehyden, p-Isopropyl- bzw. p-Tertiärbutyl-benzaldehyd, die durch Aldolkondensation mit Propionaldehyd und anschliessender relativ schwierig durchführbarer Partialhydrierung (vgl. US-PS 3 520 934, US-PS 3 520 935, US-PS 3 280 192, DE-AS 12 80 835) in die gewünschten Verbindungen überführt werden können.

Demgegenüber ermöglicht das erfindungsgemässe Verfahren, dass man diese als Riechstoffe sehr interessanten substituierten Phenylpropanale aus dem ebenso kostengünstigen wie auch gut zugänglichen Benzaldehyd in bekannter Weise durch Umsetzung mit Propionaldehyd zum α-Methylzimtaldehyd (3-Phenyl-2-methyl-prop-2-enal), Hydrierung zum α-Methyl-dihydrozimtalkohol (3-Phenyl-2-methyl-propanol), erfindungsgemässer Alkylierung und anschliessend in bekannter Weise durch Dehydrierung des erhaltenen substituierten Phenylpropanals in guten Ausbeuten und aus billigen Grundprodukten erhalten kann. Alle angeführten Syntheseschritte sind technisch einfach und problemlos durchführbar.

Es ist bekannt, dass Alkylaromaten durch Umsetzung von Aromaten mit Olefinen oder Alkylhalogeniden (G. Olah «Friedel-Crafts and Related Reactions», Band II, S. 3 ff, S. 417 ff) in Gegenwart von stark sauren Katalysatoren, wie z.B. Schwefelsäure, Phosphorsäure erhalten werden können. So führt beispielsweise die Umsetzung von Toluol mit Isobutylen in guter Ausbeute zum p-tert.-Butyl-toluol.

Setzt man statt Toluol Aromaten mit längeren Alkylketten wie Äthylbenzol oder Propylbenzol ein, so verläuft die Umsetzung deutlich langsamer (R.N. Volker und S.V. Zavgorodnig, Dokl. Akad. Nauk S.S.S.R. 133, 843 (1960).

Ausserdem war es allgemein bekannt (s. Olah cit. Seite 477 ff. bzw. 641 f), dass aromatische Verbindungen nach Friedel-Crafts auch mittels Alkanolen oder Estern anstelle von Olefinen oder Halogenverbindungen alkyliert werden können. Im Hinblick darauf ist es als überraschend zu bezeichnen, dass die nachstehend näher beschriebene Alkylierung der Hydroxyalkylbenzole und der Ester dieser Hydroxyalkylbenzole der Formel II in glatter Reaktion zu hohen Ausbeuten an den gewünschten Verfahrensprodukten I führt, da eine Selbstkondensation der Verbindungen der Formel II zumindest in einem solchen Ausmass zu befürchten war, dass die Reaktion für ein technisches Verfahren nicht brauchbar ist.

Entgegen dieser Befürchtung wurde gefunden, dass auch Aromaten, die den eingangs definierten Rest $-CH_2-CHR^2-CH_2OX$ tragen, unter bestimmten Bedingungen glatt und in guten Ausbeuten mit Olefinen bzw. Alkylhalogeniden alkyliert werden können.

Gegenstand der vorliegenden Erfindung ist dementsprechend ein Verfahren zur Herstellung von in p-Stellung substituierten Phenylpropanolen und deren Estern der allgemeinen Formel I

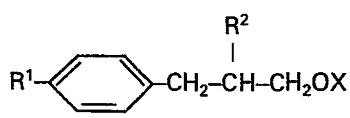

(I)

in der $R^1$ einen Alkylrest mit 3 bis 10 C-Atomen oder einen Cycloalkylrest mit 5 bis 7 C-Atomen, $R^2$ H oder einen Alkylrest mit 1 bis 4 C-Atomen und X Wasserstoff oder einen Acylrest mit 2 bis 10 C-Atomen bedeuten, das dadurch gekennzeichnet ist, dass man Phenylpropanole oder deren Ester der allgemeinen Formel II

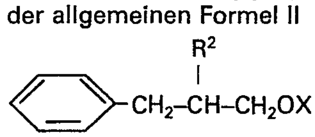

(II)

in Gegenwart von Lewis-Säuren mit einem Halogenid der Formel III

$R^1$–Hal (III)

in der Hal für Cl, Br oder J steht, oder dem Olefin, das durch Eliminierung von H-Hal aus dem Halogenid der Formel III abgeleitet werden kann, umsetzt.

Als Ausgangsstoffe der Formel II kommen im wesentlichen die im folgenden genannten Phenylpropanole bzw. Phenylpropanolester in Betracht:

3-Phenyl-propanol, 3-Phenyl-2-methyl-propanol, 3-Phenyl-2-ethyl-propanol, 3-Phenyl-2-isopropyl-propanol, 3-Phenyl-2-methyl-propylacetat, 3-Phenyl-2-methyl-propylpropionat, 3-Phenyl-2-methyl-propylbutyrat und 3-Phenyl-2-methyl- propylisobutyrat.

Die Ausgangsprodukte sind bekannte Verbindungen, die technisch auf relativ einfache Weise durch Aldolkondensation des gut zugänglichen Benzaldehyds mit dem entsprechenden aliphatischen Aldehyd, anschliessende Hydrierung der bei der Aldolkondensation gebildeten Phenylpropenale unter üblichen Bedingungen und ggf. Veresterung der Phenylpropanole nach bekannten Methoden erhalten werden können.

Als Halogenide der Formel III können folgende Verbindungen verwendet werden: n-Propylchlorid, Isopropylchlorid, Isopropylbromid, n-Butylchlorid, sek.-Butylchlorid, Isobutylchlorid, t-Butylchlorid, Isoamylchlorid, t-Amylchlorid, t-Amylbromid, Cyclopentylchlorid, Cyclohexylchlorid und Cyclohexylbromid.

Alternativ können anstelle der Halogenide folgende Olefine· als Ausgangsstoffe eingesetzt werden:

Propen, 2-Methyl-prop-1-en (Isobutylen), n-But-1-en, n-But-2-en, n-Pent-1-en, n-Pent-2-en, 2-Methyl-but-2-en, 2-Methyl-but-1-en, 2,3-Dimethyl-but-2-en, n-Hex-1-en, n-Hept-1-en, Cyclopenten, Cyclohexen und Cyclohepten.

Als Alkylierungskatalysatoren kommen Lewis-Säuren wie $AlCl_3$, $AlBr_3$, $TiCl_4$, $Zr\ Cl_4$, $V\ Cl_3$, $SnCl_4$, $ZnCl_2$, $FeCl_3$, $FeBr_3$, $BF_3$, $BF_3 \cdot CH_3COOH$ oder $BF_3 \cdot H_3PO_4$ in Betracht. Bevorzugt werden $FeCl_3$, $FeBr_3$, $BF_3$ und $BF_3$-Additionsverbindungen.

Die Lewis-Säuren verwendet man im allgemeinen in Mengen von 5 bis 200, vorzugsweise 25 bis 150 Gew.-%, bezogen auf die Ausgangsverbindung der Formel II.

Die Umsetzung wird in der Regel bei Temperaturen zwischen 0 und 150 °C, vorzugsweise zwischen 10 und 100 °C durchgeführt. Es kann lösungsmittelfrei oder in Anwesenheit von Lösungsmitteln, die unter den Reaktionsbedingungen inert sind, gearbeitet werden.

Als Lösungsmittel sind beispielsweise geeignet: 1,2-Dichlorethan, 1,2-Dichlorpropan, Methylenchlorid, Chloroform, Tetrachlorkohlenstoff, Chlorbenzol, m-Dichlorbenzol, Cyclohexan, Nitrobenzol und Schwefelkohlenstoff.

Zur Durchführung der Reaktion geht man im allgemeinen so vor, dass man die Ausgangsverbindung II bzw. deren Lösung in einem Lösungsmittel unter Kühlung mit der Lewis-Säure versetzt, das erhaltene Gemisch auf die Reaktionstemperatur erhitzt und hierein bei Reaktionstemperatur das Halogenid der Formel III oder das entsprechende Olefin langsam einträgt. Die Aufarbeitung des Reaktionsgemisches erfolgt auf übliche Weise durch Eintragen in Wasser und destillative Aufarbeitung der organischen Phase.

Die Phenylpropanole der Formel I können nach dem erfindungsgemässen Verfahren entweder direkt oder aus den entsprechenden Estern hergestellt werden. Zur Herstellung der Phenylpropanole der Formel I aus den entsprechenden Estern wendet man mit Vorteil die Methode der Umesterung mit einem niederen Alkanol wie Methanol oder Ethanol an.

Mit Hilfe des erfindungsgemässen Verfahrens gelingt es, die Phenylpropanole bzw. -propanolester der Formel I auf technisch einfache Weise und mit guten Ausbeuten aus gut zugänglichen Ausgangsstoffen herzustellen. Die hergestellten Verbindungen sind selbst als Riechstoffe interessant und ausserdem Schlüsselprodukte für die Herstellung von begehrten Riechstoffen (siehe US-PS 1 844 013; US-PS 2 976 321; US-PS 3 280 192; US-PS 3 520 934; US-PS 3 520 935 und DE-AS 12 80 835) sowie von Pflanzenschutzmitteln (siehe DE-OS 26 56 747).

Besondere Bedeutung hat das erfindungsgemässe Verfahren im Rahmen der Gesamtsynthese der wichtigen Riechstoffe IV

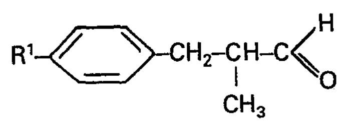
(IV)

(darunter vor allem mit $R^1$=tert.-Butyl). Man nimmt diese Synthese in der Weise vor, dass man

– Benzaldehyd in an sich bekannter Weise durch Aldolkondensation mit n-Propanal in 2-Methyl-3-phenyl-2-propenal überführt,

– das erhaltene 2-Methyl-3-phenyl-2-propenal in an sich bekannter Weise zu 2-Methyl-3-phenyl-propanol hydriert,

– dieses gegebenenfalls in an sich bekannter Weise verestert,

– das erhaltene 2-Methyl-3-phenyl-propanol bzw. dessen Ester gemäss Anspruch 1 alkyliert und

– die erhaltenen Phenylpropanole der Formel I in an sich bkannter Weise in die entsprechenden Aldehyde der Formel IV überführt.

Beispiel 1

103 g 3-Phenyl-2-methyl-propylacetat wurden in 350 g Cyclohexan gelöst, wonach diese Lösung unter Kühlung mit 81 g wassarfreiem Eisen-III-chlorid versetzt und anschliessend auf 70 °C erhitzt wurde. Bei dieser Temperatur wurden in die Reaktionsmischung unter starkem Rühren 40 g Isobutylen eingeleitet. Anschliessend liess man das Reaktionsgemisch auf Raumtemperatur abkühlen, rührte mit 500 g Wasser aus, filtrierte und arbeitete die organische Phase des erhaltenen Filtrats destillativ auf. Man erhielt 112 g 3-p-tert.-Butylphenyl-2-methyl-propylacetat vom Siedepunkt Kp=110 °C/0,1 mbar. Die Ausbeute betrug 84%, bezogen auf eingesetztes 3-Phenyl-2-methyl-propylacetat.

Beispiel 2

Eine Lösung von 75 g 3-Phenyl-2-methyl-propanol in 350 g 1,2-Dichlorethan wurde unter Kühlung mit 89 g wasserfreiem Eisen-III-chlorid versetzt und anschliessend auf 50 °C erwärmt. Bei 50 °C wurden innerhalb einer Stunde 70 g tert.-Butylchlorid zugetropft. Anschliessend liess man das Reaktionsgemisch auf Raumtemperatur abkühlen, rührte es mit 500 g Wasser aus, filtrierte und arbeitete die organische Phase des Filtrates destillativ auf.

Diese Destillation führte zu 88,5 g 3-p-tert.-butylphenyl-2-methylpropanol vom Kp = 135 °C/3 mbar. Die Ausbeute betrug 86%, bezogen auf eingesetztes 3-Phenyl-2-methyl-propanol.

Beispiel 3

Zu einer Lösung von 99 g 3-Phenyl-2-methylpropylacetat in 200 g 1,2-Dichlorethan wurden unter Kühlung 20 ml $BF_3 \cdot H_3PO_4$ (Additionsprodukt von ortho-Phosphorsäure an Bortrifluorid) zugegeben. In dieses Gemisch wurden sodann unter intensivem Rühren bei 60 °C 31 g Isobutylen eingeleitet. Anschliessend wurde das Reaktionsgemisch auf Raumtemperatur abgekühlt und nacheinander mit 200 ml 10 gew.-%iger Natronlauge und mit 200 ml Wasser gewaschen. Die organische Phase des Reaktionsaustrages wurde danach destillativ aufgearbeitet. Man erhielt 37,6 g nichtumgesetztes 3-Phenyl-2-methyl-propylacetat vom Kp. = 77 °C/0,12 mbar zurück und 70,6 Teile 3-p-tert.-Butyl-2-methyl-propylacetat vom Kp = 110 °C/0,1 mbar. Der Umsatz betrug 62%, die Ausbeute 89%, bezogen auf umgesetztes 3-Phenyl-2-methyl-propylacetat.

Beispiel 4

Zu einer Lösung von 97 g 3-Phenyl-2-methylpropylacetat in 350 g 1,2-Dichlorethan wurden unter Kühlung 89 g wasserfreies Eisen-III-chlorid zugefügt. Bei 70 °C wurden innerhalb einer Stunde 57,5 g Cyclohexen zu der Reaktionsmischung zugetropft. Nach Abkühlung auf Raumtemperatur wurde das Reaktionsprodukt mit 500 g Wasser gewaschen und filtriert. Die organische Phase des Filtrates wurde sodann destillativ gereinigt.

Man erhielt 105 g 3-p-Cyclohexylphenyl-2-methylpropylacetat vom KP = 150 °C/0,3 mbar. Die Ausbeute betrug 76%, bezogen auf 3-Phenyl-2-methyl-propylacetat.

Beispiel 5

Auf die in Beispiel 4 beschriebene Weise, jedoch anstelle des Cyclohexens mit 47,7 g Cyclopenten, wurde das 3-p-Cyclopentylphenyl-2-methyl-propylacetat vom Kp = 138 °C/0,01 mbar hergestellt. Hierbei betrug die Ausbeute 74%, bezogen auf 3-Phenyl-2-methyl-propylacetat.

Beispiel 6

Zu einer Lösung von 316,7 g 3-Phenyl-2-methylpropylpropionat in 900 g 1,2-Dichlorethan wurden unter Kühlung 274 g wasserfreies Eisen-III-chlorid gegeben. In diese Mischung wurden bei 70 °C unter intensivem Rühren innerhalb von 2 Stunden 95 g Isobutylen eingeleitet. Anschliessend wurde auf Raumtemperatur abgekühlt und mit 1000 g Wasser ausgerührt. Das Reaktionsgemisch wurde dann filtriert und die organische Phase des Filtrates durch Destillation gereinigt. Man erhielt 340 g 3-p-tert.-Butylphenyl-2-methyl-propylpropionat vom Kp = 103 °C/0,01 mbar. Die Ausbeute betrug 84%, bezogen auf 3-Phenyl-2-methyl-propylpropionat.

Beispiel 7

Eine Lösung von 179 g 3-Phenyl-2-methyl-propyl-isobutyrat in 550 g 1,2-Dichlorethan wurde unter Kühlung mit 144 g wasserfreiem Eisen-III-chlorid versetzt. Bei 70 °C wurden innerhalb einer Stunde 51 g Isobutylen in die Reaktionsmischung eingeleitet. Anschliessend liess man auf Raumtemperatur abkühlen, rührte das Reaktionsprodukt mit 500 g Wasser aus, filtrierte und arbeitete die organische Phase des Filtrates destillativ auf. Man erhielt 192,5 g 3-p-tert.-Butyl phenyl-2-methyl-propylisobutyrat vom Kp = 119 °C/0,1 mbar. Die Ausbeute betrug 86%, bezogen auf 3-Phenyl-2-methyl-propylisobutyrat.

Beispiel für Umesterung

546 g p-tert.-Butylphenyl-2-methyl-propylacetat wurden mit 160 g Methanol und 1 g Natriummethylat versetzt und zum Sieden erhitzt. Der dabei entstehende Essigsäuremethylester wurde über eine 50 cm-Kolonne abdestilliert. Nach beendeter Reaktion (ca. 3 Stunden) wurde der Kolbenrückstand abgekühlt, in ca. 500 ml Diethyläther aufgenommen und mit Wasser neutral gewaschen. Das Lösungsmittel wurde anschliessend abdestilliert und das Reaktionsprodukt einer fraktionierten Destillation unterworfen, Kp = 135 °C/4 mbar. Die Ausbeute an p-tert.-Butylphenyl-2-methyl-propanol betrug 93% der Theorie.

**Patentansprüche**

1. Verfahren zur Herstellung von in p-Stellung substituierten Phenylpropanolen und deren Estern der allgemeinen Formel I

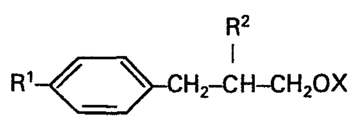

(I)

in der $R^1$ einen Alkylrest mit 3 bis 10 C-Atomen oder einen Cycloalkylrest mit 5 bis 7 C-Atomen, $R^2$ H oder einen Alkylrest mit 1 bis 4 C-Atomen und X Wasserstoff oder einen Acylrest mit 2 bis 10 C-Atomen bedeuten, dadurch gekennzeichnet, dass man Phenylpropanole und deren Ester der allgemeinen Formel II

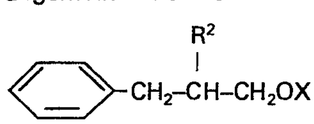

(II)

in Gegenwart von Lewis-Säuren mit einem Halogenid der Formel

$R^1$–Hal (III)

in der Hal für Cl, Br oder J steht, oder dem Olefin, das durch Eliminierung von H-Hal aus dem Halogenid der Formel III abgeleitet werden kann, umsetzt.

2. Verfahren nach Anspruch 1, dadurch gekennzeichnet, dass man als Lewis-Säure Eisen-III-chlorid, Bortrifluorid oder Additionsverbindungen des Bortrifluorids einsetzt.

3. Verfahren zur Herstellung von Duftstoffen der allgemeinen Formel IV

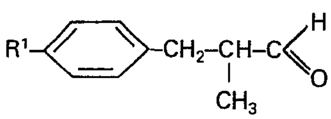
(IV)

dadurch gekennzeichnet, dass man

– Benzaldehyd in an sich bekannter Weise durch Aldolkondensation mit n-Propanal in 2-Methyl-3-phenyl-2-propenal überführt,

– das erhaltene 2-Methyl-3-phenyl-2-propenal in an sich bekannter Weise zu 2-Methyl-3-phenylpropanol hydriert,

– dieses ggf. in an sich bekannter Weise verestert,

– das erhaltene 2-Methyl-3-phenyl-propanol bzw. dessen Ester gemäss Anspruch 1 alkyliert und

– die erhaltenen Phenylpropanole der Formel I in an sich bekannter Weise in die entsprechenden Aldehyde der Formel IV überführt.

**Revendications**

1. Procédé de préparation de phénylpropanols bustitués en position para et de leurs esters de formule générale I

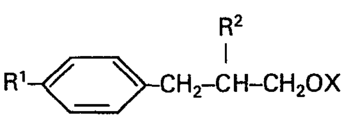
(I)

dans laquelle $R^1$ représente un groupe alkyle en C3–C10 ou un groupe cycloakyle en C5–C7, $R^2$ représente H ou un groupe alkyle en C1–C4 et X représente l'hydrogène ou un radical acyle en C2–C10, caractérisé en ce que l'on fait réagir des phénylpropanols et leurs esters de formule générale II

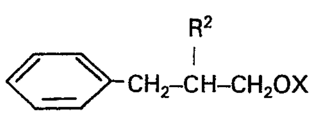
(II)

en présente d'acides de Lewis avec un halogénure de formule

$R^1$–Hal (III)

dans laquelle Hal représente Cl, Br ou J, ou avec l'oléfine dérivant de l'halogénure de formule III par élimination de H-Hal.

2. Procédé selon la revendication 1, caractérisé en ce que l'on utilise en tant qu'acide de Lewis de chlorure de fer-III, le trifluorure de bore ou des composés d'addition du trifluorure de bore.

3. Procédé de préparation de matières aromatique de formule générale IV

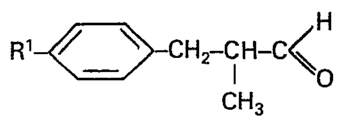
(IV)

caractérisé en ce que

– on convertit le benzaldéhyde de manière connue en soi, par condensation aldolique avec le n-propanal, en 2-méthyl-3-phényl-2-propénal,

– on hydrogène le 2-méthyl-3-phényl-2-propénal obtenu, de manière connue en soi, en le 2-méthyl-3-phényl-propanol,

– on estérifie éventuellement ce dernier de manière connue en soi,

– on alkyle le 2-méthyl-3-phényl-propanol ou son ester obtenu selon la revendication 1 et

– on convertit les phénylpropanols obtenus de formule I, de manière connue en soi, en les aldéhydes correspondants de formule IV.

**Claims**

1. A process for the preparation of p-substituted phenylpropanols and esters thereof of the general formula I

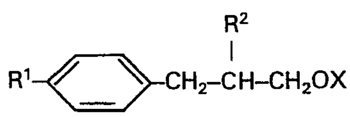
(I)

where $R^1$ is alkyl of 3 to 10 carbon atoms or cycloalkyl of 5 to 7 carbon atoms, $R^2$ is H or alkyl of 1 to 4 carbon atoms, and X is hydrogen or acyl of 2 to 10 carbon atoms, wherein phenylpropanols and esters thereof of the general formula II

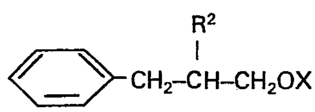
(II)

are reacted in the presence of Lewis acids with a halide of the formula

$R^1$–hal (III),

where hal is Cl, Br or J, or the olefin that can be derived by elimination of H-hal from the halide of the formula III.

2. A process as claimed in claim 1, wherein iron(III) chloride, boron trifluoride or an adduct of boron trifluoride is used as Lewis acid.

3. A process for the production of fragrances of the general formula IV

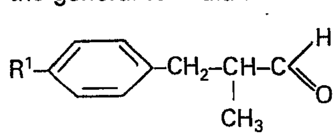
(IV)

wherein

– benzaldehyde is converted into 2-methyl-3-phenyl-2-propenal in a conventional manner by aldol condensation with n-propanal,

– the resulting 2-methyl-3-phenyl-2-propenal is hydrogenated to 2-methyl-3-phenylpropanol in a conventional manner,

– if desired, the latter is esterified in a conventional manner,

– the resulting 2-methyl-3-phenylpropanol or ester thereof is alkylated according to claim 1, and

– the resulting phenylpropanols of the formula I are converted into the corresponding aldehydes of the formula IV in a conventional manner.